# EUROPEAN PATENT APPLICATION

(11) **EP 1 273 664 A1**
(43) Date of publication of application: **08.01.2003**
(21) Application number: 00913066.7
(22) Date of filing: 31.03.2000
(51) Int. Cl.: C12P 7/64, C12N 1/19

(54) **PROCESS FOR PRODUCING LIPIDS AND LIPID-SECRETING MICROORGANISMS**

(71) Applicant: IDEMITSU PETROCHEMICAL CO., LTD., Tokyo 108-0014 (JP)
(72) Inventor: SHIMAUCHI, Toshitsugu, Sodegaura-shi, Chiba 299-0293 (JP); NAKAJIMA, Toshiaki, Tokyo 108-0041 (JP)
(74) Representative: Bannerman, David Gardner
(86) International application number: JP0002128
(87) International publication number: WO01075136

(57) **Abstract**

A method for producing a lipid comprising cultivating

A lipid such as a free fatty acid, ergosterol or ubiquinone is produced by cultivating a bacterium, preferably a mutant or recombinant strain of a yeast belonging to the genus Saccharomyces, in which an intracellular lipid content of the parent strain of the bacterium is not more than 0.17 g/g dry cells, and an extracellular lipid secretion of the bacterium is not less than 0.15 g/L, and accumulating the lipid in the medium.

## Description

### TECHNICAL FIELD

The present invention relates to a method for producing lipids and more particularly to a method for producing lipids using microorganisms secreting free fatty acids to the outside of the microorganism cells.

### BACKGROUND ART

As the method for producing an oil and fat by a fermentation method using a microorganism has been known a method in which yeast belonging to the genus *Saccharomyces* is used. For example, a method for producing a lipid component (including triglycerides) having a high palmitoleic acid content using a *Saccharomyces* yeast (JP 63-287491 A) and an intracellular production of palmitoleic acid using a *Saccharomyces* yeast, etc. are known (JP 62-289191 A).

Since the *Saccharomyces* yeast accumulates an oil and fat in the microbial cells, a step of extracting an oil and fat from the microbial cells becomes necessary. This step includes separation of microbial cells from the culture broth, disruption of microbial cells, and extraction of an oil and fat with solvents, or extraction of an oil and fat from dry microbial cells by using solvents, and so on, and is complicated and inefficient.

On the other hand, as a method for producing an oil and fat outside the cells of microorganisms, a method of cultivating fungi or algae in the presence of a surfactant (JP 62-3791 A) has been known.

Also, there have been proposed a method in which a microorganism belonging to the genus *Trichosporon, Saccharomycopsis, Candida* or *Cryptococcus* is cultivated in the presence of a fatty acid alkyl ester or a fatty acid to produce an oil and fat outside the microbial cells (JP 5-91889 A); and a method of producing an oil and fat cells characterized in that a microorganism belonging to the genus *Trichosporon* and having an ability of producing an oil and fat outside the microbial cells by being cultivated in the presence of a carbohydrate is cultivated in a medium containing a carbohydrate to form and accumulate an oil and fat in the culture broth outside the microbial cells and the formed and accumulated oil and fat are collected from the culture broth (JP 7-236492 A). The oils and fats produced outside the microbial cells in these methods are triglycerides and no free fatty acids are secreted.

Furthermore, a method in which a long chain fatty acid is produced outside the microbial cells by using a mutant of *Candida lypolytica* is used (Miyagawa, et al., Agric. Biol. Chem., 48, 499 (1984)) has been known. Note that *Candida lypolytica* is now classified as the genus *Yarrowia*. The above-mentioned mutant is a strain obtained by subjecting a parent strain that is deficient in acyl-CoA synthetase and cannot decompose fatty acid through β-oxidation to mutation treatment, screening a strain showing a low cell density by Percoll density gradient centrifugation, and overlaying the selected strain on a fatty-acid requiring mutant and screening a strain that supports the growth of the mutant. The resulting strain produces fatty acid in an amount above 1 mg/1 ml under optimal conditions.

However, there exists in *Saccharomyces* yeasts no strain that substantially secretes free fatty acid.

### DISCLOSURE OF THE INVENTION

An object of the present invention is to provide a method for producing lipids such as free fatty acids by using a microorganism, in particular *Saccharomyces* yeast, that secretes lipids, in particular free fatty acids.

The inventors of the present invention have made extensive studies in order to solve the above-mentioned problems and as a result, they have found that a lipid secreting yeast can be selected by fractionating a yeast cultivated in a low nitrogen source culture medium and collecting a strain separated in the high specific gravity fraction and that a yeast that is deficient in a long chain acyl-CoA synthetase, in particular *Saccharomyces cerevisiae*, efficiently produces free fatty acids, thereby achieving the present invention.

That is, the present invention is as follows.
(1) A method for producing a lipid comprising cultivating a bacterium in a liquid medium to accumulate the lipid in the medium, wherein the bacterium is a mutant or recombinant strain, an intracellular lipid content of the parent strain of the bacterium is not more than 0.17 g/g dry cells, and the extracellular lipid secretion of the bacterium is not less than 0.15 g/L.
(2) The method according to (1), wherein the microorganism is a yeast.
(3) The method according to (1) or (2), wherein the lipid is selected from the group consisting of free fatty acids, ergosterols or ubiquinones.
(4) The method according to any one of (1) to (3), wherein the mutant strain is a strain separated as a high specific gravity fraction by fractionating microbial cells cultivated in a medium having a carbon source content of not less than 0.8% in terms of carbon and a nitrogen source content of not more than 0.002% in terms of nitrogen, by density gradient centrifugation.
(5) The method according to any one of (1) to (4), wherein the mutant or recombinant is a strain deficient in a long-chain acyl-CoA synthetase.
(6) The method according to any one of (1) to (5), wherein the yeast belongs to the genus *Saccharomyces.*
(7) The method according to (6), wherein the yeast is *Saccharomyces cerevisiae.*
(8) The method according to any one of (1) to (7), wherein the liquid medium contains a surfactant.
(9) A method for producing a lipid comprising cultivating a yeast which belongs to the genus *Saccharomyces* and which is deficient in a long-chain acyl-CoA synthetase in a liquid medium to accumulate the lipid in the medium.
(10) A recombinant strain of *Saccharomyces cerevisiae*, whose gene encoding a long-chain acyl-CoA synthetase has been disrupted.
(11) A method for producing a lipid-secreting microorganism, characterized by subjecting a microorganism to mutation treatment, cultivating the microorganism in a medium having a carbon source content of not less than 0.8% in terms of carbon and a nitrogen source content of not more than 0.002% in terms of nitrogen, fractionating the obtained microbial cells by density gradient centrifugation, selecting strains separated as a high specific gravity fraction, and further selecting out of the selected strains a strain secreting a lipid to the outside of the cells.

Hereinafter, the present invention will be described in detail.

The method for producing a lipid according to the present invention is characterized in that a bacterium is used, wherein the bacterium is a mutant or recombinant strain, an intracellular lipid content of the parent strain of the bacterium is not more than 0.17 g/g dry cells, and the extracellular lipid secretion of the bacterium is not less than 0.15 g/L.

The term "lipid" in the present invention means fatty acid, sterols or ubiquinones, etc. but it does not include triglycerides. Specifically, fatty acids such as palmitic acid, palmitoleic acid, stearic acid, oleic aid, linolic acid, α-linolenic acid, γ-linolenic acid, and dihomo-γ-rinolenic acid, sterols such as ergosterol, etc. ubiquinones such as coenzyme Q6, and the like may be mentioned. Note that the lipids produced by the present invention may contain triglycerides so far as they contain the above-mentioned lipids.

The amount of extracellular lipid secretion is measured typically in the following manner. A yeast cell is inoculated in 200 ml of a medium A described later on and cultivated at 30°C for 72 hours. After completion of the cultivation, wet cells are separated by centrifugation, and after 100 ml of sterile distilled water is added to the wet cells and mixed, centrifugation is performed again to wash the cells. The resultant cells are inoculated into 100 ml of a medium B described later on so as to become about 5 g/L as dry cells and cultivated at 30°C for 96 hours. After completion of the cultivation, the culture broth is separated into cells and supernatant by centrifugation. Then, total lipid is extracted from the supernatant by using 30 ml of chloroform. Also, it is desirable that the operation of extraction is repeated several times. Dehydration from the organic solvent layer is performed by adding 30 g of anhydrous sodium sulfate to the extracted liquid and stirring, followed by filtration through a filter. From the filtrate chloroform is removed by using an evaporator to collect extracellular lipid and the weight is measured. The lipid in the obtained extract can be analyzed by gas chromatography or liquid chromatography.

The intracellular lipid content is measured typically in the following manner. After suspending 2 g of the cell obtained in the same manner as described above in 20 ml of distilled water, 60 ml of chloroform/methanol (1/2, vol/vol) is added and the cells are disrupted. The cell-disrupted liquid is filtered to separate an oil-containing liquid. To the filtrate are added 20 ml of chloroform and 20 ml of distilled water followed by mixing. Thereafter, from the two-layer-separated liquid was separated the lower layer (organic solvent layer) to extract the intracellular lipid. It is preferred that the cell disruption and the extraction operations as described above are repeated several times. The weight of the lipid extract thus obtained is measured.

The mutant of the yeast used in the present invention can be obtained by subjecting a yeast to ordinary mutation treatment, for example, a chemical treatment with a mutagen such as ethyl methanesulfonate (EMS) or N-methyl-N'-nitro-N-nitrosoguanidine (NTG), or a physical treatment such as UV irradiation and selecting a mutant that comes to secrete a lipid. The mutant that secretes a lipid can be selected by cultivating the yeast subjected to the mutation treatment in a low nitrogen source medium, for example, a medium having a carbon source content of not less than 0.8% in terms of carbon atoms and a nitrogen source content of not more than 0.002% in terms of nitrogen atoms, fractionating the obtained cells by density gradient centrifugation, and selecting a strain separated as a high specific gravity fraction.

Furthermore, a lipid-secreting strain is obtained by forming colonies of the selected candidate strain on an agar medium, overlaying an agar medium having suspended therein an oleic acid-requiring mutant, and separating a strain that grows around the oleic acid-requiring mutant strain. Note that the mutant used in the present invention may be a spontaneous mutant.

As described above, the inventors of the present invention have found that a lipid-secreting strain can be selected by fractionating yeast cells cultivated in a specific medium by specific gravity. This is believed to be attributable to a large specific gravity since cultivation in a low nitrogen source medium as described above makes a yeast that inherently produces no excessive lipid produce excessive lipid and since it is harder for the lipid-secreting strain to accumulate a lipid such as a fatty acid in the cell than the non-secreting strain.

As described above, a lipid-secreting yeast can be produced.

The lipid-secreting strains specifically includes a mutant or recombinant strain that is deficient in long-chain acyl-CoA synthetase (EC 6.2.1.3). As will be described later on, cloning a gene that revert the mutation of the lipid secreting mutant obtained in Example 1 indicated that the gene has the same sequence as the known FAA1 gene.

The yeast used in the present invention may be a recombinant of the yeast that is deficient in long-chain acyl-CoA synthetase. The recombinant deficient in long-chain acyl-CoA synthetase specifically includes a strain of which a chromosomal gene encoding the long-chain acyl-CoA synthetase (FAA1) has been disrupted (FAA1 gene-disrupted strain). The disruption of the FAA1 gene can be obtained by using a vector which is not autonomously replicable and which is to be introduced by incorporation into a chromosome, for example, YIp type vector such as YIp5, and inserting in the vector an FAA1 gene from which a portion of the sequence is deleted, and the obtained recombinant vector is transformed to a host yeast. That is, the recombinant vector is incorporated in the host cells and the deletion-type FAA1 gene and the vector DNA are inserted in the sequence of the FAA1 gene on the chromosome DNA by homologous recombination between the FAA1 gene on the chromosome DNA and the deletion-type FAA1 gene in the recombinant vector. As a result, the sequence of FAA1 on the chromosome is disrupted, so that no active long-chain acyl-CoA synthetase will be expressed. The gene-disrupted strain can be selected usually by the phenotype of a marker gene contained in the vector. Also, the fact that the FAA1 gene on the chromosome has been disrupted can be confirmed by preparing the chromosome DNA of a candidate strain, and performing PCR by using a suitable oligonucleotide primers or by performing Southern hybridization by using as a suitable oligonucleotide or an FAA1 gene fragment.

The microorganism used in the present invention includes yeasts preferably having an intracellular lipid content in a parent strain of not more than 0.17 g/g dry cells, for example, *Saccharomyces* yeasts such as *Saccharomyces cerevisiae* and *Saccharomyces carlsbergensis; Shizosacoharomyces* yeasts; Schwanniomyces yeasts; *Kloeckera* yeasts; *Candida* yeasts (*Candida utilis*); etc. According to the present invention, as in these yeasts, lipids can be efficiently produced without using high oil and fat-producing yeasts such as *Candida lypolytica* and *Lipomyces starkeyi.*

The techniques for preparation of chromosome DNA, PCR, Southern hybridization, etc. are well known and described in, for example, Molecular Cloning; A Laboratory Manual (Maniatis, et al., Cold spring Harbor Laboratory, Cold Spring Harbor Press (1989)), etc. Also, cultivation, transformation, etc., of yeasts are likewise well known and described in Ito, et al., (1983) J. Bacteriol., 153, 163-168, etc.

The media for cultivating mutants or recombinants of yeast that secrete lipids are not particularly limited and various media containing a carbon source, a nitrogen source, inorganic ions, etc. may be used. Specifically, production media described later on are used.

As the main carbon source may be mentioned sugars such as glucose, sucrose and fructose; alcohols such as ethanol and glycerol; amino acids such as glutamic acid and aspartic acid; etc. Also, as the nitrogen source may be mentioned ammonia, ammonium sulfate, ammonium acetate, ammonium nitrate, ammonium chloride, yeast extracts, meat extracts, peptone, casein hydrolysates, corn steep liquor, etc. As the inorganic ions may be mentioned potassium ion, magnesium ion, iron ion, manganese ion, sodium ion, zinc ion, copper ion, molybdenum ion, iodine ion, etc.

It is preferred that surfactants such as polyoxyethylene (20) cetyl ether (Brij-58), and a polyglycol ether (nonionic) surfactant (for example, Tergitol NP-40) are added in an amount of 0.01 to 1.0%.

The cultivation conditions may be set as appropriate depending on the kind of yeast to be used. For example, in the case of Saccharomyces cerevisiae, cultivation is performed under aerobic conditions at 20 to 35°C and 50 to 300 rpm for 1 to 7 days. Note that for producing lipids, it is preferable to use microbial cells in a stationary phase rather than microbial cells in a logarithmic phase. The more preferred are the microbial cells that have entered a stationary phase due to the deficiency of the nitrogen source since they use the carbon source in the medium for the synthesis of lipids so that yield of lipids to sugars is increased.

As a preferred medium and cultivation condition, high extracellular lipid productivity is shown in a cultivation in which microbial cells cultivated in, for example, a medium obtained by changing the amount of ammonium sulfate in the low nitrogen source medium described later on to 0.05%, YPD medium or the high nitrogen source medium described later on is transferred to a low nitrogen source medium.

For collecting the lipids from the culture broth, unnecessary substances such as microbial cells are removed from the culture broth by centrifugation, filtration or the like, and the lipids are extracted from the thus obtained supernatant with suitable solvents. As the solvents may be mentioned halogenated lower alkanes such as chloroform, methylene chloride, carbon tetrachloride, and 1,2-dichloroethane; aromatic hydrocarbons such as benzene and xylene; etc. The amount of the solvent is not particularly limited so far as it is an amount in which the lipids can be sufficiently extracted; usually it is 100 ml to 1 liter per 1 liter of the supernatant.

The extracted lipids are used as they are or after further purification depending on the purpose. For the purification of lipids or separation of a specific lipid, ordinary lipid separation and purification methods such as TLC, liquid chromatography, etc. are only needed to be performed.

### BRIEF DESCRIPTION OF THE DRAWINGS

Fig. 1 illustrates a restriction map of SHC39 and a construction of a plasmid SHC39-2 in which the inserted fragment of SHC 39 is subcloned; and
Fig. 2 illustrates construction of a plasmid for disrupting FAA1 gene;

### BEST MODE FOR CARRYING OUT THE INVENTION

Hereinafter, the present invention will be described in more detail by way of examples.

The compositions of the medium, etc. used in the present examples are as follows.

| (1) YPD medium | |
|---|---|
| Glucose | 2% |
| Polypeptone | 2% |
| Yeast extract | 1% |

| (2) YPD plate medium | |
|---|---|
| Glucose | 2% |
| Polypeptone | 2% |
| Yeast extract | 1% |
| Agar | 2% |

| (3) High-nitrogen medium | |
|---|---|
| Glucose | 2% |
| Yeast nitrogen base | 0.17% |
| (not including ammonium sulfate, amino acid) (Difco Co.) | |
| Ammonium sulfate | 0.5% |
| Casamino acids | 0.5% |
| Uracil | 0.002% |
| Tryptophan | 0.005% |

| (4) Low-nitrogen medium | |
|---|---|
| Glucose | 2% |
| Yeast nitrogen base | 0.17% |
| (not including ammonium sulfate, amino acid) | |
| (Difco Co.) | |
| Ammonium sulfate | 0.01% |
| Leucine | 0.01% |
| Uracil | 0.002% |
| Tryptophan | 0.005% |

| (5) Soft agar medium(I) | |
|---|---|
| Glucose | 2% |
| Polypeptone | 2% |
| Yeast extract | 1% |
| Polyoxyethylene(20)cetyl ether(Brij-58) | 0.5% |
| Agar | 0.75% |

| (6) Soft agar medium(II) | |
|---|---|
| Glucose | 2% |
| Polypeptone | 0.5% |
| Yeast extract | 0.1% |
| KH₂PO₄ | 0.1% |
| MgSO₄ | 0.005% |
| Polyoxyethylene(20)cetyl ether(Brij-58) | 0.5% |
| Agar | 0.75% |

| (7) Medium A | |
|---|---|
| Glucose | 2% |
| Polypeptone | 2% |
| Yeast extract | 1% |
| Glycerol | 2% |
| Tergitol NP-40 | 1% |

| (8) Medium B | |
|---|---|
| Glucose | 3% |
| Yeast nitrogen base | 0.17% |
| (not including ammonium sulfate, amino acid) | |
| (Difco Co.) | |
| Ammonium sulfate | 0.01% |
| Leucine | 0.01% |
| Uracil | 0.002% |
| Tryptophan | 0.005% |
| Tergitol NP-40 | 1% |

| (9) EMS solution | |
|---|---|
| EMS (ethyl methanesulfonate) | 0.3ml |
| 0.2M phosphoric acid buffer (pH8.0) | 9.2ml |
| 40% glucose solution | 0.5ml |

### Example 1 Isolation of a fatty acid-secreting mutant of Saccharomyces cerevisiae

*Saccharomyces cerevisiae* CG378 strain (obtained from American Type Culture Collection (ATCC 204664)) was cultured in 10 ml of YPD medium at 30°C and 100 rpm for 16 hours with shaking. After completion of the cultivation, the cells were collected by centrifugation (3,000 rpm, 5 minutes). The microbial cells were suspended in 5 ml of a 0.2 M phosphate buffer solution (pH 8.0) and collected by centrifugation (3,000 rpm, 5 minutes), thereby washing the microbial cells. Thereafter, the microbial cells were suspended in 10 ml of an EMS solution and gently shaken at 30°C for 30 minutes to perform a mutation treatment. To the EMS treated liquid was added 9.8 ml of a 6% sodium hyposulfite solution and the resultant was left to stand for 10 minutes to stop the mutation treatment. The above-mentioned mutation treatment targeted a survival rate of 15% as a guideline.

The microbial cells subjected to the mutation treatment as described above were collected by centrifugation (3,000 rpm, 5 minutes) and inoculated to 50 ml of a high nitrogen source medium and shake cultured at 30°C and 100 rpm for 16 hours. After the cultivation, the microbial cells were washed by repeating the collection of cells by centrifugation (3,000 rpm, 5 minutes) and suspension in 5 ml of a 0.2 M phosphate buffer solution (pH 8.0) twice. Then, the microbial cells were inoculated in 50 ml of a low nitrogen medium and shake cultured at 30°C and at 100 rpm for 16 hours. The microbial cells were collected by centrifugation (3,000 rpm, 5 minutes) and suspended in a 2% glucose solution (cell concentration 2×10⁸ cell/ml). The suspension (0.2 ml) was overlaid on the top of a centrifugation tube in which 0.25 M sucrose solutions having dissolved therein Percoll (Pharmacia) to 100%, 90%, 80%, and 70%, respectively, were overlaid in the order cited and density gradient centrifugation was performed at 5,000×g for 5 hours. After the centrifugation, the layer of microbial cells having high specific gravity was separated. The operation described above was repeated three times.

The high specific gravity strain obtained as described above was coated on a YPD plate medium and cultivated at 30°C for 3 days. After colony formation, UV irradiation was performed for 15 minutes by a 15 W sterilizing lamp placed at a distance of 50 cm. Then, on the plate medium was overlaid 10 ml of a suspension soft agar medium (I) containing 10⁵ cells/ml of an oleic acid-requiring mutant (KD115 strain, available from American Type Culture Collection (Accession Number ATCC 204991)) and a mutant around which the oleic acid-requiring mutant had grown and formed a halo was separated. The mutant was named "SFS-1 strain".

The SFS-1 strain was internationally deposited at National Institute of Bioscience and Human-Technology, Research Institute of Industrial Science and Technology, Ministry of International Trade and Industry at (1-3, Higashi 1-chome, Tsukuba-shi, Ibaraki-ken, Japan, Postal Code: 305-8566) on March 17, 2000 under and assigned Accession Number FERM BP-7096.

### Example 2 Analysis of Lipid Secreted by Mutant

The mutant obtained in Example 1 was inoculated in 200 ml of medium A and shake cultured at 30°C and at 200 rpm for 72 hours. After completion of the cultivation, wet microbial cells were separated by centrifugation (2,400×g, 10 minutes). To the wet microbial cells was added 100 ml of sterilized distilled water, and after mixing, the resultant was again centrifuged (the same as above) to wash the microbial cells. The microbial cells were inoculated in 100 ml of medium B to be about 5 g/L as dry microbial cells and shake cultured at 30°C and at 200 rpm for 96 hours. After completion of the cultivation, the microbial cells and supernatant in the culture broth were separated by centrifugation (the same as above), and the supernatant was extracted with 30 ml of chloroform three times (in total 90 ml). The chloroform was evaporated to concentrate the extract and the extract was separated by TLC. The TLC was performed by using silica gel 70 plate (Wako) and development was performed with n-hexane:diethyl ether:acetic acid = 80:30:1. Coloring the thin layer with iodine indicated two spots. Each of the spots was recovered and analyzed by gas chromatography (for fatty acid analysis, FFAP/5% Uniport S 60/80; for sterol analysis, Silicone 0V-172 Uniport HP 60/80) which resulted in detection of fatty acids (palmitic acid, palmitoleic acid, stearic acid, and oleic acid) and ergosterol. Also, the analysis of the extract by liquid chromatography (column, ODS; mobile layer, methanol/ethanol = 65/35; flow rate, 1.0 ml/minute; detection, UV detector (275 nm)) resulted in detection of ubiquinone (coenzyme Q₆). Note that the triglycerides and ergosterol esters present in the microbial cells of *Saccharomyces cerevisiae* was not detected.

### Comparative Example 1 Analysis of Lipid Secreted by Parent Strain

Parent strain (CG378 strain) that was not subjected to mutation treatment was analyzed for the fatty acids in the chloroform extract of the supernatant by TLC in the same manner as in Example 2. As a result, lipids containing free fatty acids and ergosterol were not detected.

### Example 3 Quantitation of Lipids Produced by Mutant - (1)

### <1> Quantitation of Intracellular Lipids

In 200 ml of medium A was inoculated the above-mentioned mutant (SFS-1 strain) and cultivated at 30°C for 72 hours. After completion of the cultivation, wet microbial cells were separated by centrifugation (2,400×g, 10 minutes). To the wet microbial cells was added 100 ml of sterilized distilled water, and after mixing, the resultant was again centrifuged (the same as above) to wash the microbial cells. The microbial cells were inoculated in 100 ml of medium B to be about 5 g/L as dry microbial cells and cultivated at 30°C and for 96 hours. After completion of the cultivation, wet microbial cells were separated by centrifugation (2,400×g, 10 minutes).

From the wet microbial cells obtained as described above was separated exactly a 2 g portion, which was suspended in 20 ml of distilled water, and then the microbial cells were disrupted by using a homogenizer to which 60 ml of chloroform/methanol (1/2, vol/vol) and 40 ml of glass beads (diameter 0.40 to 0.50 mm) were added (4 minutes, 4,000 rpm). From the microbial cells/glass beads mixed liquid was separated an oil-containing solution by using a filter (Qualitative Filter No. 2, produced by Advantec Toyo Kaisha, Ltd.). To the separated filtrate were added 20 ml of chloroform and 20 ml of distilled water and was mixed, and then the lower layer (organic solvent layer) was separated from the two-layer-separated liquid to extract intracellular lipids. The operations of microbial cells disruption with chloroform/methanol and chloroform extraction were repeated additional two times (total 3 times) to extract total lipid from the microbial cells.

To the organic solvent layer (containing oil) extracted as described above was added 30 g of anhydrous sodium sulfate and was stirred, and then the mixture was filtered by using a filter (the same as above) to perform dehydration from the organic solvent layer. From the filtrate intracellular lipids were obtained by removing chloroform and methanol by using an evaporator and the weight was measured.

On the other hand, an exactly 2 g portion was likewise separated from the above-mentioned wet microbial cells and heated at 105°C for 3 hours to remove the moisture and dry microbial cell weight was measured.

From the intracellular lipid weight and dry microbial cell weight measured in the manner described above, a lipid content per unit of dry microbial cell weight was calculated. As a result, the intracellular lipid content was 0.055 mg/g dry microbial cells and microbial cells yield (dry microbial cells) was 5.87 g/L. From these, the total lipid yield in the microbial cells was 0.32 g/L.

### <2> Quantitation of Extracellular Lipid Secretion Content

The lipid-secreting mutant (SFS-1 strain) was inoculated in 200 ml of the medium A and cultivated at 30°C for 72 hours. The cultivation itself was the same as in <1> above. After cultivating in the medium A for 72 hours, the microbial cells were washed and cultivated in the medium B for 96 hours. After completion of the cultivation, the microbial cells and supernatant were separated by centrifugation (2,400×g, 10 minutes). After adding 30 ml of chloroform to the supernatant and stirring, the chloroform layer was separated. This chloroform extraction was repeated additional two times (thus three times in total), and extraction of total lipid from the supernatant was performed. After adding 30 g of anhydrous sodium sulfate to the extracted liquid and stirring, the mixture was filtered by using a filter (Qualitative Filter No. 2, produced by Advantec Toyo Kaisha, Ltd.), thereby performing dehydration from the organic solvent layer. From the filtrate, chloroform was removed by using an evaporator to obtain extracellular lipids and the weight was measured. As a result, the extracellular lipid production was 0.76 g/L. Also, the lipid secretion content per dry microbial cells was 0.13 g/g dry microbial cells. The above results are shown in Table 1.

TLC analysis of the obtained intracellular and extracellular lipids performed in the same manner as in Example 2 resulted in detection of monoglycerides, diglycerides, triglycerides (major component), ergosterol, ergosterol esters and fatty acids as the intracellular lipids and ergosterol and fatty acids as extracellular lipids. From the fact that no glyceride was detected in the extracellular lipids, it is apparent that the secretion of lipids is not due to bacteriolysis.

### Comparative Example 2 Quantitation of Lipids Produced by Parent Strain - (1)

Parent strain (CG378 strain) that was not subjected to mutation treatments was quantitated on intracellular lipid content and extracellular lipid secretion content in the same manner as in Example 3. As a result, the intracellular lipid content was 0.163 g/g dry microbial cells and microbial cells yield (dry microbial cells) was 8.16 g/L. From these, the total lipid yield in the microbial cells was 1.33 g/L. Also, the extracellular lipid production was 0.012 g/L. Also, the lipid secretion content per dry microbial cells was 0.0015 g/g dry microbial cells. The results are shown in Table 1.

### Example 4 Quantitation of Lipids Produced by Mutant - (2)

In 300 ml of the medium A was inoculated the above-mentioned mutant (SFS-1 strain) and cultivated at 30°C for 96 hours. After completion of the cultivation, wet microbial cells were separated by centrifugation (2,400×g, 10 minutes). For a 2-g portion of the wet microbial cells, intracellular lipid content and content of extracellular lipid secretion were quantitated in the same manner as in Example 3.

As a result, the intracellular lipid content was 0.052 g/g dry microbial cells and microbial cells yield (dry microbial cells) was 1.98 g/L. From these, the total lipid yield in the microbial cells was 0.10 g/L. Also, the extracellular lipid production was 0.175 g/L. Also, the lipid secretion content per dry microbial cells was 0.088 g/g dry microbial cells. The results are shown in Table 2.

### Comparative Example 3 Quantitation of Lipids Produced by Parent Strain - (2)

Parent strain (CG378 strain) that was not subjected to mutation treatments was quantitated on intracellular lipid content and content of extracellular lipid secretion in the same manner as in Example 4. As a result, the intracellular lipid content was 0.062 g/g dry microbial cells and microbial cells yield (dry microbial cells) was 12.2 g/L. From these the total lipid yield in the microbial cells was 1.33 g/L. Also, the extracellular lipid production was 0.005 g/L. Also, the lipid secretion content per dry microbial cells was 0.0004 g/g dry microbial cells. The results are shown in Table 2.

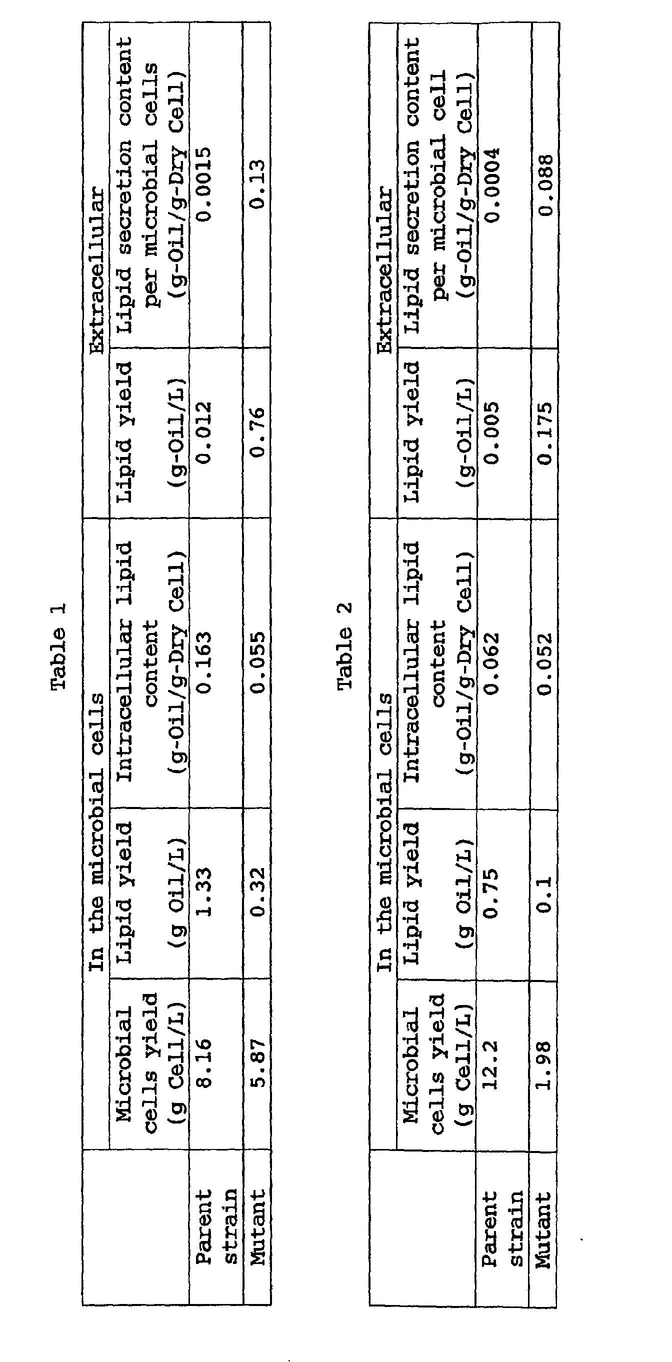

### Example 5 Identification of Gene Participating in Secretion of Lipids

### <1> Preparation of DNA Library

From Saccharomyces cerevisiae X-2180-1A strain, which was a laboratory yeast that showed no lipids-secreting property, was prepared genomic DNA in accordance with the Philippsen, et al. method (Methods in Enzymology, vol. 194, p169-182 (1991)). The strain is available from American Type Culture Collection (Accession No. ATCC 26786).

After partially decomposing the thus obtained genomic DNA with restriction enzyme Sau3AI, 5 to 10 kbp DNA fragments were fractionated by a sucrose density gradient ultracentrifugation method and ligated to yeast-*Escherichia coli* shuttle vector YEp24 cleaved with BamHI (Lundblad et al., Current Protocols in Molecular Biology, Vol. 2, unit 13.4 (1989)) to obtain a recombinant vector. *Escherichia coli* JM109 was transformed with the recombinant vector, transformed strains having acquired ampicillin resistance were selected and recombinant DNA was recovered from the transformed strains to obtain a DNA library.

### <2> Introduction of Library into Lipid Secreting Mutant

To the lipid-secreting mutant (SFS-1) obtained in Example 1 was introduced the above-mentioned DNA library in accordance with the lithium acetate method described in Ito, et al., J. Bacteriol., 153:163-168 (1983). Specifically, it was performed as follows.

The mutant (SFS-1 strain) was cultivated in 50 ml of YPD medium at 30°C for 12 hours. After collecting the cells by centrifugation (2,400×g, 5 minutes), they were suspended in 50 ml of a TE buffer solution (10 mM Tris-HCl. 1 mM EDTA) and again centrifuged to make them into a pellet. The pellet was resuspended in about 1 ml of a TE buffer solution, and to a 0.5 ml portion was added 0.2 M lithium acetate, followed by gently shaking the suspension at 30°C for 1 hour. After the shaking, 10 µl of the DNA library (DNA content about 10 µg) was added to the lithium acetate/microbial cells mixed solution, which was left to stand at 30°C for 30 minutes.

Then, polyethylene glycol 4000 was added so as to have a final concentration of about 35% (w/v) and mixed. The cell liquid was maintained at 30°C for 2.5 hours and then the cells were collected by centrifugation (2,400×g, 5 minutes). The microbial cells pellet obtained by centrifugation was suspended in 0.2 ml of a TE buffer solution (the same as above) and a 0.1 ml portion was inoculated by spreading it over a synthetic plate medium containing no uracil and cultivated at 30°C by stationary cultivation for 3 days, thereby obtaining a transformant of the mutant to which the library DNA was introduced and from which the uracil requirement was lost.

### <3> Selection of Gene Reverting Lipid Secretion Mutation

From the transformants obtained as described above was selected a candidate strain that retains a gene that revert the lipid secretion mutation to the original by the method of overlaying a soft agar containing an oleic acid requiring strain described in Miyagawa, et al., Agric. Biol. Chem., 48(2), 499-503 (1984).

The transformants were inoculated in a synthetic plate medium containing no uracil to have about 100 colonies/plate medium and subjected to stationary culture at 30°C for 3 days to form colonies. The plate medium was subjected to a UV treatment (UV germicidal lamp of 15 W, UV being irradiated at a distance of 30 cm for 15 minutes) to sterilize the microbes on the surface of the colonies. To the plate medium was overlaid 5 ml of soft agar medium (II) containing 10⁵ cells of oleic acid-requiring *Saccharomyces cerevisiae* KD115 strain (ATCC 204991), which was cultivated at 30°C for 2 days by stationary cultivation.

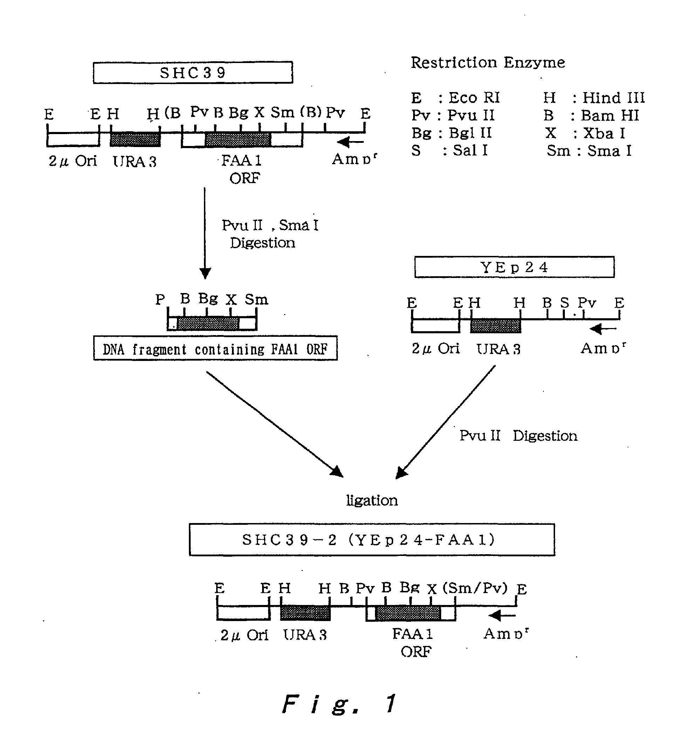

Around most of the colonies of the transformants were formed haloes by the growth of the KD115 strain but a colony with no halo formed was found. From this colony was extracted total DNA in the same manner as described above and the DNA was introduced into *Escherichia coli* to obtain a clone resistant to ampicillin. From this clone was recovered a plasmid, which was named SHC39. The restriction enzyme cleavage map of SHC39 is shown in Fig. 1.

Then, to confine the essential region in the DNA fragment inserted in SHC39, an about 6 kB DNA fragment obtained by cleaving the plasmid (SHC39) with restriction enzymes PvuII and SmaI was inserted into the PvuII site of YEp24 to obtain a plasmid SHC39-2. The process is illustrated in Fig. 1.

Introduction of SHC39-2 into a lipid-secreting mutant and study of its lipid-secreting property resulted in inhibition of the lipid-secreting property of the transformant of the mutant. This revealed that the gene reverting the lipid secretion mutation exists in the DNA fragment inserted in SHC39-2 (PvuII-SmaI).

The base sequence of the inserted DNA in SHC39-2 was determined by a conventional dideoxy method. Prior to the dideoxy method, the inserted portion was treated with exonuclease III and mung bean nuclease to render it short-chained by the methods described in Gene, vol. 28, 351-359 (1984) and Gene, vol. 33, 103-119 (1985), respectively, to prepare various clones in which a portion of the inserted fragment was lost and which have various chain lengths. For this process, a deletion kit for kilo sequence (Takara Shuzo Co., Ltd.) was used.

For the various types of clones thus obtained, the DNA base sequence was determined on the inserted fragments thereof by the dideoxy method (Science, vol. 214, 1205-1210 (1981)) utilizing fluorescent primer (Bio-Techniques, vol. 5, 754-765 (1987)). For the determination of base sequences, a DNA sequencer (Applied Biosystems, Inc., ABI373) was used. The results are shown SEQ ID NO:1.

### <4> Identification of Gene Reverting Lipid Secretion Mutation

The base sequence shown in SEQ ID NO:1 contains an open reading frame of 2,139 bp, which was believed to encode a protein consisting of 713 amino acids (SEQ ID NO:2). On this base sequence, a homologous base sequence was retrieved by the FASTA method by Lipman, et al. (Science, vol. 227, 1435-1441 (1985)). The results indicate that the base sequence concerned completely coincided with the base sequence of the long-chain acyl-CoA synthetase gene (FAA1) of the yeast *Saccharomyces cerevisiae*.

The above finding suggested that in *Saccharomyces cerevisiae*, the FAA1 gene is a gene that controls the secretion of lipids. Note that SHC39-2 was renamed as YEp24-FAA1.

### Example 6

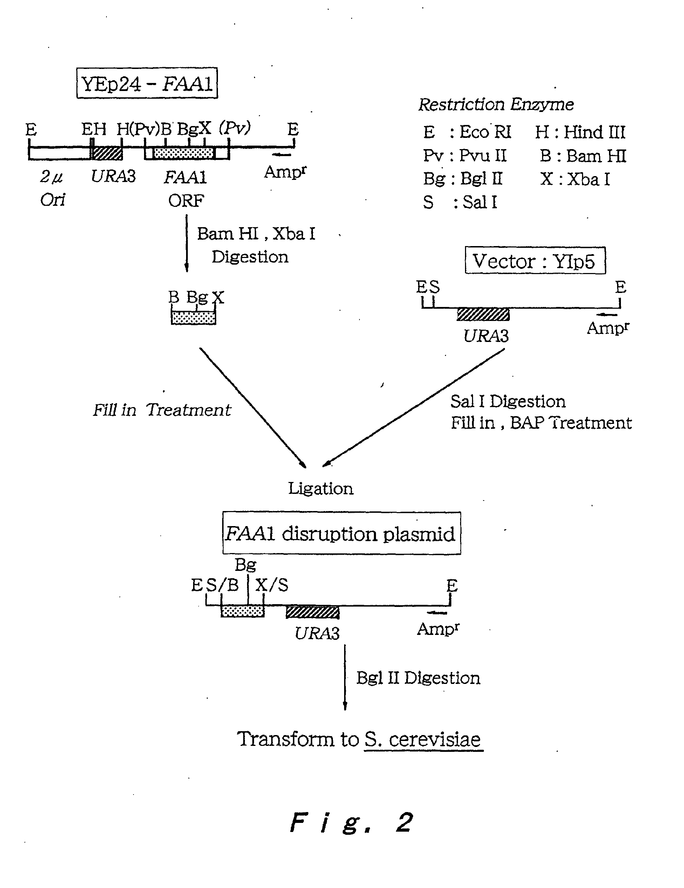

YEp24-FAA1 containing the FAA1 gene was cleaved with BamHI and XbaI to cleave out an FAA1 gene fragment in which the 5'- and 3'-terminal regions were lost and both the termini were blunted by using a DNA blunting kit (Takara Shuzo Co., Ltd.). On the other hand, plasmid vector YIp5 was cleaved with SalI and blunted and then dephosphorylated with BAP (bacterial alkaline phosphatase), followed by ligating the resultant with the above-mentioned FAA1 fragment (Fig. 2).

*Saccharomyces cerevisiae* CG378 strain was transformed with the plasmid for disrupting FAA1 gene obtained as described above and transformants were selected by using recovery of uracil source prototrophy as an index. Furthermore, a chromosome DNA was prepared from the obtained transformant and subjected to Southern hybridization to select a strain of which the FAA1 gene has been disrupted.

The chloroform extract of the supernatant of the FAA1 gene disrupted strain obtained as described above was analyzed by TLC in the same manner as in Example 1. As a result, free fatty acids and ergosterol were detected.

### INDUSTRIAL APPLICABILITY

By the present invention, lipids can be efficiently produced by using yeasts. Also, breeding and selection of yeast strains suitable for the production of lipids becomes possible.

## Claims

1. A method for producing a lipid comprising cultivating a bacterium in a liquid medium to accumulate the lipid in the medium, wherein the bacterium is a mutant or recombinant strain, an intracellular lipid content of the parent strain of the bacterium is not more than 0.17 g/g dry cells, and the extracellular lipid secretion of the bacterium is not less than 0.15 g/L.

2. The method according to claim 1, wherein the microorganism is a yeast.

3. The method according to claim 1 or 2, wherein the lipid is selected from the group consisting of free fatty acids, ergosterols and ubiquinones.

4. The method according to any one of claims 1 to 3, wherein the mutant strain is a strain separated as a high specific gravity fraction by fractionating microbial cells cultivated in a medium having a carbon source content of not less than 0.8% in terms of carbon and a nitrogen source content of not more than 0.002% in terms of nitrogen, by density gradient centrifugation.

5. The method according to any one of claims 1 to 4, wherein the mutant or recombinant strain is a strain deficient in a long-chain acyl-CoA synthetase.

6. The method according to any one of claims 1 to 5, wherein the yeast belongs to the genus *Saccharomyces.*

7. The method according to claim 6, wherein the yeast is *Saccharomyces cerevisiae.*

8. The method according to any one of claims 1 to 7, wherein the liquid medium contains a surfactant.

9. A method for producing a lipid comprising cultivating a yeast which belongs to the genus *Saccharomyces* and which is deficient in a long-chain acyl-CoA synthetase in a liquid medium to accumulate the lipid in the medium.

10. A recombinant strain of *Saccharomyces cerevisiae,* whose gene encoding a long-chain acyl-CoA synthetase has been disrupted.

11. A method for producing a lipid-secreting microorganism, comprising:
subjecting a microorganism to mutation treatment;
cultivating the microorganism in a medium having a carbon source content of not less than 0.8% in terms of carbon and a nitrogen source content of not more than 0.002% in terms of nitrogen;
fractionating the obtained microbial cells by density gradient centrifugation;
selecting strains separated as a high specific gravity fraction; and
further selecting out of the selected strains a strain secreting a lipid to the outside of the cells.


SEQUENCE LISTING

<110> IDEMITSU PETROCHEMICAL CO., LTD.

<120> METHOD FOR PRODUCING LIPIDS AND
      LIPID-SECRETING MICROORGANISMS

<130> H110440P992

<140>
<141> 2000-03-31

<160> 2

<170> PatentIn Ver. 2.0

<210> 1
<211> 2892
<212> DNA
<213> Saccharomyces cerevisiae

<220>
<221> CDS
<222> (625)..(2724)

<400> 1
cagctgctta cgctgcggaa agtaaacaga cctagctagc ggaccgatag agccggctga 60
actttataac aaatgcgcct tctaacaagc gatgaagcca tgcttagcgc ggcaaagaga 120
attacttaga atatggatga tgcagccctg tcatattctt ccataaggga atgacaagga 180

caatagtaaa gtcgatcacg ccctgtcagg aacatgatct ccgccatgtg aatatttatg 240

catgatacag gcacgaatac atcatataat acgaatgaca caggggcacc cacccatcgc 300

atatcaggag aacttccctg tgcatacggg acagcagaga taggctgttc tcgcgcgttt 360

tttttttttt tttgcttttt ttggttcctt tcgcggatat cgtattatcg atattacaac 420

aaaaatgttt ggagatctcc ttcgctgtct ttaagtaaaa ggctttaaac cgcttatttt 480

tctctctttt tttcctttta cccctcactt gtcgtgagac aattgtagta tatttagtaa 540

gtatagaaag ttcttgttgt taaaaaactc gttaggatac aataaaaact agaacaaaca 600

caaaagacaa aaaaagacaa caat atg gtt gct caa tat acc gtt cca gtt 651

Met Val Ala Gln Tyr Thr Val Pro Val

1               5

ggg aaa gcc gcc aat gag cat gaa act gct cca aga aga aat tat caa 699

Gly Lys Ala Ala Asn Glu His Glu Thr Ala Pro Arg Arg Asn Tyr Gln

10                  15                  20                  25

tgc cgc gag aag ccg ctc gtc aga ccg cct aac aca aag tgt tcc act 747

Cys Arg Glu Lys Pro Leu Val Arg Pro Pro Asn Thr Lys Cys Ser Thr

                30                  35                  40

gtt tat gag ttt gtt cta gag tgc ttt cag aag aac aaa aat tca aat 795

Val Tyr Glu Phe Val Leu Glu Cys Phe Gln Lys Asn Lys Asn Ser Asn

            45                  50                  55

gct atg ggt tgg agg gat gtt aag gaa att cat gaa gaa tcc aaa tcg 843

Ala Met Gly Trp Arg Asp Val Lys Glu Ile His Glu Glu Ser Lys Ser

        60                  65                  70

gtt atg aaa aaa gtt gat ggc aag gag act tca gtg gaa aag aaa tgg 891

Val Met Lys Lys Val Asp Gly Lys Glu Thr Ser Val Glu Lys Lys Trp

    75                  80                  85

atg tat tat gaa cta tcg cat tat cat tat aat tca ttt gac caa ttg 939

Met Tyr Tyr Glu Leu Ser His Tyr His Tyr Asn Ser Phe Asp Gln Leu

90                  95                  100                 105

acc gat atc atg cat gaa att ggt cgt ggg ttg gtg aaa ata gga tta 987

Thr Asp Ile Met His Glu Ile Gly Arg Gly Leu Val Lys Ile Gly Leu
                110                 115                 120
aag cct aat gat gat gac aaa tta cat ctt tac gca gcc act tct cac 1035
Lys Pro Asn Asp Asp Asp Lys Leu His Leu Tyr Ala Ala Thr Ser His
            125                 130                 135
aag tgg atg aag atg ttc tta gga gcg cag tct caa ggt att cct gtc 1083
Lys Trp Met Lys Met Phe Leu Gly Ala Gln Ser Gln Gly Ile Pro Val
        140                 145                 150
gtc act gcc tac gat act ttg gga gag aaa ggg cta att cat tct ttg 1131
Val Thr Ala Tyr Asp Thr Leu Gly Glu Lys Gly Leu Ile His Ser Leu
    155                 160                 165
gtg caa acg ggg tct aag gcc att ttt acc gat aac tct tta tta cca 1179
Val Gln Thr Gly Ser Lys Ala Ile Phe Thr Asp Asn Ser Leu Leu Pro
170                 175                 180                 185
tcc ttg atc aaa cca gtg caa gcc gct caa gac gta aaa tac ata att 1227
Ser Leu Ile Lys Pro Val Gln Ala Ala Gln Asp Val Lys Tyr Ile Ile
                190                 195                 200
cat ttc gat tcc atc agt tct gag gac agg agg caa agt ggt aag atc 1275
His Phe Asp Ser Ile Ser Ser Glu Asp Arg Arg Gln Ser Gly Lys Ile
            205                 210                 215
tat caa tct gct cat gat gcc atc aac aga att aaa gaa gtt aga cct 1323
Tyr Gln Ser Ala His Asp Ala Ile Asn Arg Ile Lys Glu Val Arg Pro
        220                 225                 230
gat atc aag acc ttt agc ttt gac gac atc ttg aag cta ggt aaa gaa 1371
Asp Ile Lys Thr Phe Ser Phe Asp Asp Ile Leu Lys Leu Gly Lys Glu
    235                 240                 245
tcc tgt aac gaa atc gat gtt cat cca cct ggc aag gat gat ctt tgt 1419
Ser Cys Asn Glu Ile Asp Val His Pro Pro Gly Lys Asp Asp Leu Cys
250                 255                 260                 265

tgc atc atg tat acg tct ggt tct aca ggt gag cca aag ggt gtt gtc 1467
Cys Ile Met Tyr Thr Ser Gly Ser Thr Gly Glu Pro Lys Gly Val Val
                270                 275                 280
ttg aaa cat tca aat gtt gtc gca ggt gtt ggt ggt gca agt ttg aat 1515
Leu Lys His Ser Asn Val Val Ala Gly Val Gly Gly Ala Ser Leu Asn
            285                 290                 295
gtt ttg aag ttt gtg ggc aat acc gac cgt gtt atc tgt ttt ttg cca 1563
Val Leu Lys Phe Val Gly Asn Thr Asp Arg Val Ile Cys Phe Leu Pro
        300                 305                 310
cta gct cat att ttt gaa ttg gtt ttc gaa cta ttg tcc ttt tat tgg 1611
Leu Ala His Ile Phe Glu Leu Val Phe Glu Leu Leu Ser Phe Tyr Trp
    315                 320                 325
ggg gcc tgc att ggt tat gcc acc gta aaa act tta act agc agc tct 1659
Gly Ala Cys Ile Gly Tyr Ala Thr Val Lys Thr Leu Thr Ser Ser Ser
330                 335                 340                 345
gtg aga aat tgt caa ggt gat ttg caa gaa ttc aag ccc aca atc atg 1707
Val Arg Asn Cys Gln Gly Asp Leu Gln Glu Phe Lys Pro Thr Ile Met
                350                 355                 360
gtt ggt gtc gcc gct gtt tgg gaa aca gtg aga aaa ggg atc tta aac 1755
Val Gly Val Ala Ala Val Trp Glu Thr Val Arg Lys Gly Ile Leu Asn
            365                 370                 375
caa att gat aat ttg ccc ttc ctc acc aag aaa atc ttc tgg acc gcg 1803
Gln Ile Asp Asn Leu Pro Phe Leu Thr Lys Lys Ile Phe Trp Thr Ala
        380                 385                 390
tat aat acc aag ttg aac atg caa cgt ctc cac atc cct ggt ggc ggc 1851
Tyr Asn Thr Lys Leu Asn Met Gln Arg Leu His Ile Pro Gly Gly Gly
    395                 400                 405
gcc tta gga aac ttg gtt ttc aaa aaa atc aga act gcc aca ggt ggc 1899
Ala Leu Gly Asn Leu Val Phe Lys Lys Ile Arg Thr Ala Thr Gly Gly

410                 415                 420                 425
caa tta aga tat ttg tta aac ggt ggt tct cca atc agt cgg gat gct 1947
Gln Leu Arg Tyr Leu Leu Asn Gly Gly Ser Pro Ile Ser Arg Asp Ala
                430                 435                 440
cag gaa ttc atc aca aat tta atc tgc cct atg ctt att ggt tac ggt 1995
Gln Glu Phe Ile Thr Asn Leu Ile Cys Pro Met Leu Ile Gly Tyr Gly
            445                 450                 455
tta acc gag aca tgc gct agt acc acc atc ttg gat cct gct aat ttt 2043
Leu Thr Glu Thr Cys Ala Ser Thr Thr Ile Leu Asp Pro Ala Asn Phe
        460                 465                 470
gaa ctc ggc gtc gct ggt gac cta aca ggt tgt gtt acc gtc aaa cta 2091
Glu Leu Gly Val Ala Gly Asp Leu Thr Gly Cys Val Thr Val Lys Leu
    475                 480                 485
gtt gat gtt gaa gaa tta ggt tat ttt gct aaa aac aac caa ggt gaa 2139
Val Asp Val Glu Glu Leu Gly Tyr Phe Ala Lys Asn Asn Gln Gly Glu
490                 495                 500                 505
gtt tgg atc aca ggt gcc aat gtc acg cct gaa tat tat aag aat gag 2187
Val Trp Ile Thr Gly Ala Asn Val Thr Pro Glu Tyr Tyr Lys Asn Glu
                510                 515                 520
gaa gaa act tct caa gct tta aca agc gat ggt tgg ttc aag acc ggt 2235
Glu Glu Thr Ser Gln Ala Leu Thr Ser Asp Gly Trp Phe Lys Thr Gly
            525                 530                 535
gac atc ggt gaa tgg gaa gca aat ggc cat ttg aaa ata att gac agg 2283
Asp Ile Gly Glu Trp Glu Ala Asn Gly His Leu Lys Ile Ile Asp Arg
        540                 545                 550
aag aaa aac ttg gtc aaa aca atg aac ggt gaa tat atc gca ctc gag 2331
Lys Lys Asn Leu Val Lys Thr Met Asn Gly Glu Tyr Ile Ala Leu Glu
    555                 560                 565
aaa tta gag tcc gtt tac aga tct aac gaa tat gtt gct aac att tgt 2379

Lys Leu Glu Ser Val Tyr Arg Ser Asn Glu Tyr Val Ala Asn Ile Cys
570                 575                 580                 585
gtt tat gcc gac caa tct aag act aag cca gtt ggt att att gta cca    2427
Val Tyr Ala Asp Gln Ser Lys Thr Lys Pro Val Gly Ile Ile Val Pro
                590                 595                 600
aat cat gct cca tta acg aag ctt gct aaa aag ttg gga att atg gaa    2475
Asn His Ala Pro Leu Thr Lys Leu Ala Lys Lys Leu Gly Ile Met Glu
            605                 610                 615
caa aaa gac agt tca att aat atc gaa aat tat ttg gag gat gca aaa    2523
Gln Lys Asp Ser Ser Ile Asn Ile Glu Asn Tyr Leu Glu Asp Ala Lys
        620                 625                 630
ttg att aaa gct gtt tat tct gat ctt ttg aag aca ggt aaa gac caa    2571
Leu Ile Lys Ala Val Tyr Ser Asp Leu Leu Lys Thr Gly Lys Asp Gln
    635                 640                 645
ggt ttg gtt ggc att gaa tta cta gca ggc ata gtg ttc ttt gac ggc    2619
Gly Leu Val Gly Ile Glu Leu Leu Ala Gly Ile Val Phe Phe Asp Gly
650                 655                 660                 665
gaa tgg act cca caa aac ggt ttt gtt acg tcc gct cag aaa ttg aaa    2667
Glu Trp Thr Pro Gln Asn Gly Phe Val Thr Ser Ala Gln Lys Leu Lys
                670                 675                 680
aga aaa gac att ttg aat gct gtc aaa gat aaa gtt gac gcc gtt tat    2715
Arg Lys Asp Ile Leu Asn Ala Val Lys Asp Lys Val Asp Ala Val Tyr
            685                 690                 695
agt tcg tct taatggatca acatttccat gataggaaag cctcatcata            2764
Ser Ser Ser
        700
ctaaagcact ttttcagttt tttgctttag aactgctacc aattataaaa tagaatgcac 2824
aggttatctt gtttggtttg ttattatcct tttttcttct ttaccagtca tatcacctca 2884
ttcacgta                                                          2892

<210> 2

<211> 700

<212> PRT

<213> Saccharomyces cerevisiae

<400> 2

Met Val Ala Gln Tyr Thr Val Pro Val Gly Lys Ala Ala Asn Glu His

1               5                   10                  15

Glu Thr Ala Pro Arg Arg Asn Tyr Gln Cys Arg Glu Lys Pro Leu Val

            20                  25                  30

Arg Pro Pro Asn Thr Lys Cys Ser Thr Val Tyr Glu Phe Val Leu Glu

        35                  40                  45

Cys Phe Gln Lys Asn Lys Asn Ser Asn Ala Met Gly Trp Arg Asp Val

    50                  55                  60

Lys Glu Ile His Glu Glu Ser Lys Ser Val Met Lys Lys Val Asp Gly

65                  70                  75                  80

Lys Glu Thr Ser Val Glu Lys Lys Trp Met Tyr Tyr Glu Leu Ser His

                85                  90                  95

Tyr His Tyr Asn Ser Phe Asp Gln Leu Thr Asp Ile Met His Glu Ile

            100                 105                 110

Gly Arg Gly Leu Val Lys Ile Gly Leu Lys Pro Asn Asp Asp Asp Lys

        115                 120                 125

Leu His Leu Tyr Ala Ala Thr Ser His Lys Trp Met Lys Met Phe Leu

    130                 135                 140

Gly Ala Gln Ser Gln Gly Ile Pro Val Val Thr Ala Tyr Asp Thr Leu

145                 150                 155                 160

Gly Glu Lys Gly Leu Ile His Ser Leu Val Gln Thr Gly Ser Lys Ala

                165                 170                 175

Ile Phe Thr Asp Asn Ser Leu Leu Pro Ser Leu Ile Lys Pro Val Gln
            180                 185                 190
Ala Ala Gln Asp Val Lys Tyr Ile Ile His Phe Asp Ser Ile Ser Ser
        195                 200                 205
Glu Asp Arg Arg Gln Ser Gly Lys Ile Tyr Gln Ser Ala His Asp Ala
    210                 215                 220
Ile Asn Arg Ile Lys Glu Val Arg Pro Asp Ile Lys Thr Phe Ser Phe
225                 230                 235                 240
Asp Asp Ile Leu Lys Leu Gly Lys Glu Ser Cys Asn Glu Ile Asp Val
                245                 250                 255
His Pro Pro Gly Lys Asp Asp Leu Cys Cys Ile Met Tyr Thr Ser Gly
            260                 265                 270
Ser Thr Gly Glu Pro Lys Gly Val Val Leu Lys His Ser Asn Val Val
        275                 280                 285
Ala Gly Val Gly Gly Ala Ser Leu Asn Val Leu Lys Phe Val Gly Asn
    290                 295                 300
Thr Asp Arg Val Ile Cys Phe Leu Pro Leu Ala His Ile Phe Glu Leu
305                 310                 315                 320
Val Phe Glu Leu Leu Ser Phe Tyr Trp Gly Ala Cys Ile Gly Tyr Ala
                325                 330                 335
Thr Val Lys Thr Leu Thr Ser Ser Ser Val Arg Asn Cys Gln Gly Asp
            340                 345                 350
Leu Gln Glu Phe Lys Pro Thr Ile Met Val Gly Val Ala Ala Val Trp
        355                 360                 365
Glu Thr Val Arg Lys Gly Ile Leu Asn Gln Ile Asp Asn Leu Pro Phe
    370                 375                 380
Leu Thr Lys Lys Ile Phe Trp Thr Ala Tyr Asn Thr Lys Leu Asn Met
385                 390                 395                 400
Gln Arg Leu His Ile Pro Gly Gly Gly Ala Leu Gly Asn Leu Val Phe

                405                 410                 415
Lys Lys Ile Arg Thr Ala Thr Gly Gly Gln Leu Arg Tyr Leu Leu Asn
            420                 425                 430
Gly Gly Ser Pro Ile Ser Arg Asp Ala Gln Glu Phe Ile Thr Asn Leu
        435                 440                 445
Ile Cys Pro Met Leu Ile Gly Tyr Gly Leu Thr Glu Thr Cys Ala Ser
    450                 455                 460
Thr Thr Ile Leu Asp Pro Ala Asn Phe Glu Leu Gly Val Ala Gly Asp
465                 470                 475                 480
Leu Thr Gly Cys Val Thr Val Lys Leu Val Asp Val Glu Glu Leu Gly
                485                 490                 495
Tyr Phe Ala Lys Asn Asn Gln Gly Glu Val Trp Ile Thr Gly Ala Asn
            500                 505                 510
Val Thr Pro Glu Tyr Tyr Lys Asn Glu Glu Glu Thr Ser Gln Ala Leu
        515                 520                 525
Thr Ser Asp Gly Trp Phe Lys Thr Gly Asp Ile Gly Glu Trp Glu Ala
    530                 535                 540
Asn Gly His Leu Lys Ile Ile Asp Arg Lys Lys Asn Leu Val Lys Thr
545                 550                 555                 560
Met Asn Gly Glu Tyr Ile Ala Leu Glu Lys Leu Glu Ser Val Tyr Arg
                565                 570                 575
Ser Asn Glu Tyr Val Ala Asn Ile Cys Val Tyr Ala Asp Gln Ser Lys
            580                 585                 590
Thr Lys Pro Val Gly Ile Ile Val Pro Asn His Ala Pro Leu Thr Lys
        595                 600                 605
Leu Ala Lys Lys Leu Gly Ile Met Glu Gln Lys Asp Ser Ser Ile Asn
    610                 615                 620
Ile Glu Asn Tyr Leu Glu Asp Ala Lys Leu Ile Lys Ala Val Tyr Ser
625                 630                 635                 640

Asp Leu Leu Lys Thr Gly Lys Asp Gln Gly Leu Val Gly Ile Glu Leu
                645                 650                 655
Leu Ala Gly Ile Val Phe Phe Asp Gly Glu Trp Thr Pro Gln Asn Gly
            660                 665                 670
Phe Val Thr Ser Ala Gln Lys Leu Lys Arg Lys Asp Ile Leu Asn Ala
        675                 680                 685
Val Lys Asp Lys Val Asp Ala Val Tyr Ser Ser Ser
    690                 695                 700